# EUROPEAN PATENT SPECIFICATION

(11) **EP 0 991 384 B1**
(45) Date of publication and mention of the grant of the patent: **22.08.2001**
(21) Application number: 98911286.7
(22) Date of filing: 27.03.1998
(51) Int. Cl.: A61F 5/455

(54) **URINATING AID FOR WOMEN**
HILFSMITTEL ZUM URINIEREN FÜR FRAUEN
APPAREIL DE MICTION DESTINE AUX FEMMES

(30) Priority: 27.03.1997 NL 1005667
(43) Date of publication of application: 12.04.2000
(73) Proprietor: Dantuma-Van Dorp, Catharina Maria, 2613 AE Delft (NL)
(72) Inventor: Dantuma-Van Dorp, Catharina Maria, 2613 AE Delft (NL)
(74) Representative: Van Breda, Jacobus
(86) International application number: NL9800176
(87) International publication number: WO9843563

(56) References cited:
- EP-A- 0 226 277
- WO-A-93/11691
- WO-A-95/07671
- NL-A- 7 512 545
- US-A- 3 964 111
- US-A- 5 370 637

## Description

The invention relates to a urinating aid for women. The invention particularly relates to such an aid which form a guidance for the urine from the vagina, from below the urethra of a woman to a place at a distance from it, without the clothing and/or the body of the woman being wetted.

In patent literature many examples of such aids can be found. The international patent application WO 93/11691, the US patent specification 5.408.703, the international patent application WO 95/07671 and the Dutch patent application 75.12545 can be mentioned. Furthermore, the US patent specification 3.964.111, the British patent application 2.002.629, the European patent application 0 226 277, the British patent application 2.240.717 and the US patent specification 5.370.637 can be referred to.

In the US patent specification 3.964.111 a urinating aid is shown, which consists of a funnel-shaped outer sleeve in which an inner lining or inner sleeve can be placed. At placing, the inner lining is entirely accommodated in the outer sleeve and can be removed after use, so that the outer sleeve can be folded and put away awaiting a next use, when a new inner lining is placed in the outer sleeve.

The urinating aid according to the invention is characterized in that the upper edge is provided with one or several elevations that are attached to the upper edge, whereby the upper edge, in view from aside on the sleeve, has a concave portion and a convex portion that merge fluently in each other, and that the convex portion is situated at the rear in the situation of use. This provides the advantage that the user's discomfort is obviated by improving the engagement to the user of the urinating aid, whilst concurrently minimizing the risk of leakage and wetting of the urinating aid's upper edge. Preferably in this respect, the concave portion and the convex portion determine a sinusoidal line.

It is further preferable that the elevations are elongated and extend in the circumferential direction of the upper edge. In this way it is prevented, especially in case of an aid consisting of one sleeve, that the upper edge of the permanent sleeve portion of the aid would contact the user's body with such a large surface, that irritation or an allergic reaction arises. Because of the elevations that surface is decreased. The elevations can have various shapes. They can be integrally formed with the upper edge or separately attached there, in which case the user is free to make use of the elevations. In case of an outer sleeve and an inner lining the elevations or projections can form a point of engagement for the inner lining.

Preferably the elevations run along elongatedly with the upper edge. They can be narrower than the upper edge. Intermediate distances between the elevations are preferably larger at the front than at the rear.

A further advantage is that the urinating aid according to the invention not only provides in keeping with the anatomic shape of the user's body, but also provides a pleasant and functional surface, that is to say the rear area of the upper edge, with which the body can be wiped after urinating.

In a further aspect of the invention the urinating aid is provided with a number of interruptions in the thickening of the upper edge, which are spaced apart in circumferential direction.

Preferably the interruptions are formed by inwardly extending, substantially V-shaped notches, in which the sleeve can be folded in radial direction into a flat state. The sleeve can then be pressed open with the fingers, the boundaries of the V-shaped notches when abutting each other forming a limit for the extent to which the sleeve can be pressed open from the flat state.

In still a further aspect of the invention the urinating aid's sleeve has an upper and a lower portion, the lower portion being movable with respect to the upper portion. In this way the user can direct and place the trickle of urine leaving the sleeve as desired. In the case of an aid according to the invention without separate inner lining the sleeve can be articulated. In the case that an inner lining is present in an outer sleeve this inner lining, as already indicated above, can extend below out of the outer sleeve. Maximum freedom of direction is obtained when the inner lining is at least flexible with the portion extending out of the outer sleeve.

In case there is an inner sleeve or second sleeve, this is preferable made of flexible, disposable, auto-biodegradable materials. The outer sleeve or, if there is no inner sleeve, the sleeve can be made of plastic material, such as polymer, particularly expanded polystyrene. The sleeve, however, can also be made of cellulose material, such as paper or cardboard.

The invention will now be elucidated on the basis of a number of exemplary embodiments shown in the accompanying drawings.

Shown is in:
Figure 1 a front view of a first embodiment of a urinating sleeve according to the invention;
Figure 2 a side view on the sleeve of Figure 1;
Figure 3 a detail of the upper edge of the sleeve of Figure 2;
Figures 3A, 3B and 3C a number of ways in which the inner lining can be connected to an outer sleeve of a urinating sleeve according to the invention;
Figure 4 a side view on a urinating sleeve according to the invention, consisting of an outer sleeve and an inner lining, which extends out of the outer sleeve;
Figures 5A, 5B and 5C a urinating sleeve according to the invention, with a special shaped upper edge;
Figures 6A, 6B and 6C some further particulars of the urinating sleeve according to the invention;
Figure 7 a blank form which a urinating sleeve according to the invention can be formed; and
Figure 8 a urinating sleeve according to the invention in the position of use.

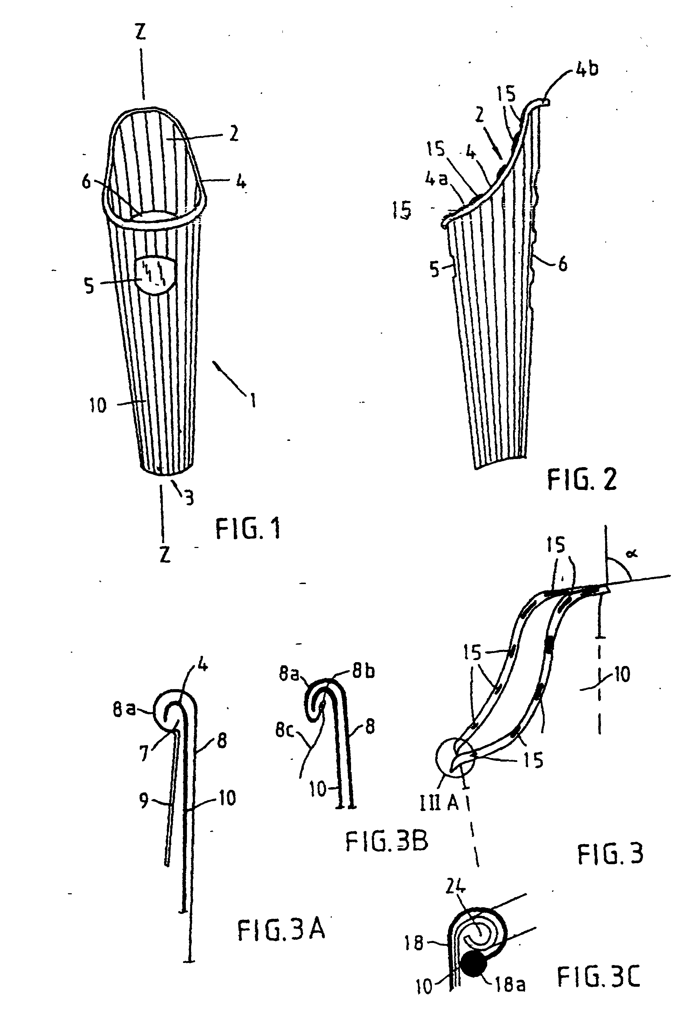

In Figure 1 a sleeve 1 of plastic material such as expanded polystyrene or cardboard is shown, which in this example mainly keeps its shape during use. The sleeve 1 has an upper end 2 and a lower end 3, which are in open connection with each other. The sleeve 1 is furthermore provided with a circumferential wall 10, which defines a passage tapering in downward direction. At the front the wall is provided with a moulded recess 5 for a thumb and at the rear provided with four moulded recesses 6 for the other fingers.

The upper edge 4 of the sleeve 1, which runs about the upper opening 2, projects out of the outer surface of the circumferential wall 10, and is for instance thickened or folded back, in which latter case a downwardly opening groove 7 (see Figure 3A and 3B) has been formed.

The upper edge 4 can, as has been indicated in Figure 2 and 3, be provided with an integrally formed or attached series of elongated elevations or hills, which are mutually spaced at distances that become larger to the front (seen in the drawing to the left). The length of the elevations 15 can decrease in the same direction. The elevations 15 are furthermore somewhat narrower than the upper edge 4.

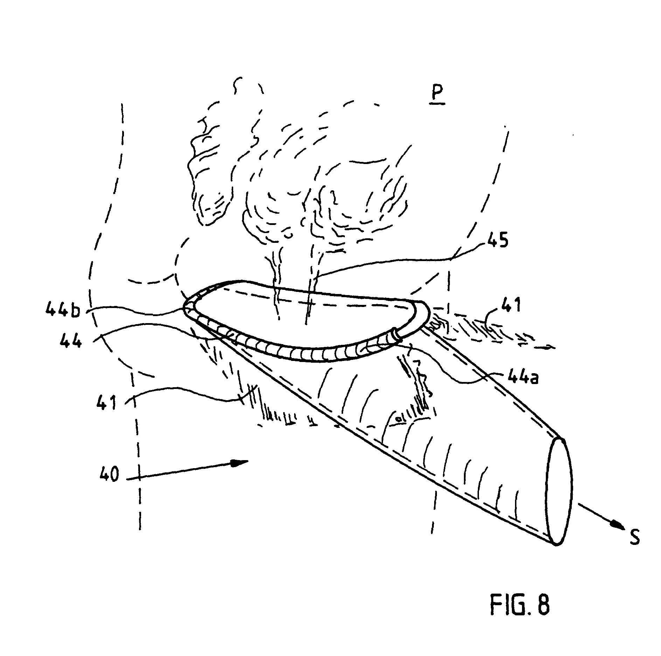

As can be seen in the Figures 2 and 3 and also in Figure 8 in side view, several portions can be discerned in the upper edges 4, 44 respectively, More to the front the upper edge portion 4a, 44a, which is concave, is situated. The upper edge portion 4a, 44a joins the upper edge portion 4b, 44b, which is convex, in rearward direction via a fluent bending point. The convex edge portion 4b, 44b ends in side view (see Figure 3) at an angle α with respect to the local generatrix of the circumferential wall 10, which can approach 90° to provide a practical help with wiping the body after urinating. In front view and in rear view, respectively, the upper edge portions which are situated on both sides of the plane of symmetry z fluently merge into one another, with a horizontal tangent.

The sleeve of Figures 1-3 can be used several times, but has to be rinsed then after each use. The sleeve 1 can also be used in connection with an inner sleeve or inner lining 8, which is made of water impermeable material and is flexible. The material preferably is bio-degradable, such as for instance bio-degradable-polyester foil.

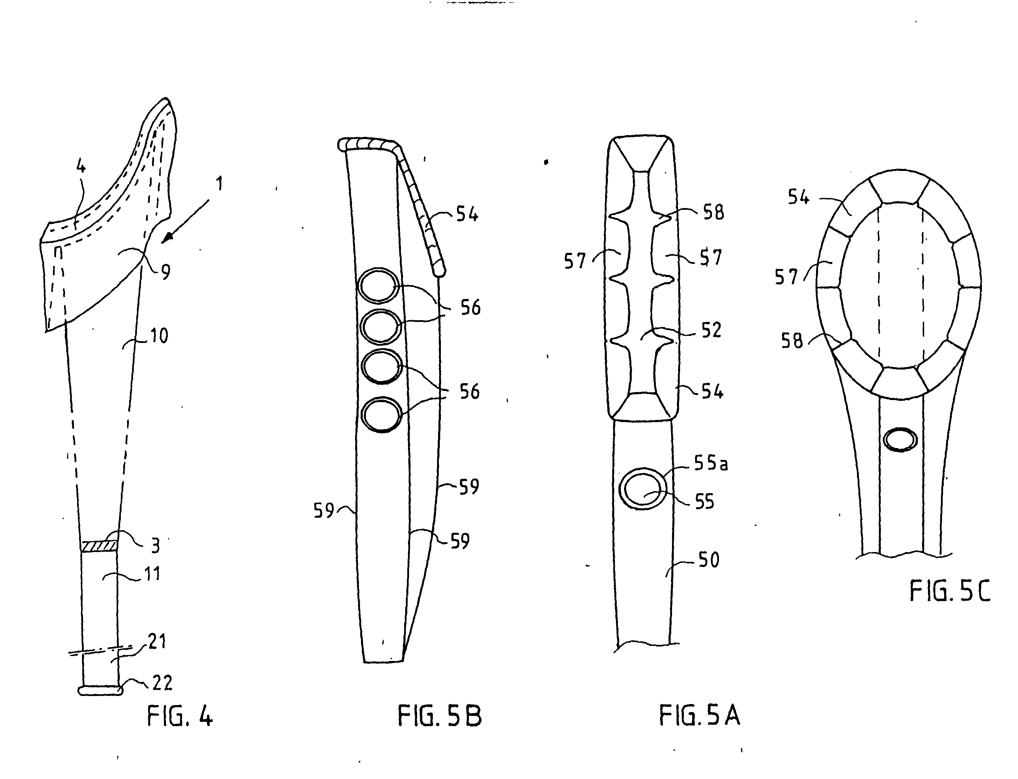

The inner lining 8 is, as can be seen in the Figures 3A, 3B and 3C, provided with an edge portion 8a which is folded back to the outside, which can fall or reach over the upper edge 4. In the representation of the Figures 3A and 4 the flanged upper edge 8a is continued in a pending skirt 9, which can be somewhat elastic to keep portion 8a taut on the upper edge 4. The edge portion 8a can alternatively, as shown in Figure 2B, be provided with a hem 8b, in which a tightening piece of string 8c is accommodated. The hem 8b and the piece of string 8c can be accommodated in the groove 7 of the upper edge 4. In Figure 3C a further alternative solution of the upper edge of the inner lining is shown, in which the inner lining 18 is provided at the top with a thickening 18a which has been formed by moulding or folding the upper edge of the inner lining 18 a number of times onto itself. The thickened portion 18a then engages over and under the upper edge 24 to keep the inner lining 18 in its place with respect to the outer sleeve 20 and to prevent sliding away.

The inner lining 8 preferably extends beyond the lower opening 3 of the sleeve 1 (Figure 4) and at the lower end preferably is provided with a weight, for instance in the form of a ring 22. The ring 22 can be relatively rigid to be more easily and securely engagable by the user at directing the projecting part 21 of the inner lining 8.

In Figure 5A an alternative urinating sleeve 50 is shown in front view and in Figure 5B in oblique rear view. The sleeve 50 is provided with a recess 55 surrounded by an elevated edge 55a at the front and at the rear with four recesses 56 surrounded by elevated edges 56a for the fingers. The upper edge 54 is formed with thickenings 57 reaching inside with flat upper sides. The thickenings are separated from one another by - in the situation of Figure 5A - V-shaped notches 58 which as it were form hinges in the edge. The sleeve 50 is made of bendable and foldable or collapsible material. To that end the sleeve 50 has four folding lines 59. The situation shown in Figure 5A is the storage state.

When the user wants to use the sleeve 50, she engages it with the fingers on the recesses 55 and 56 and exerts a slight pressure. Because of the notches 58 the upper edge can deform along until the boundaries of the notches abut one another, and the situation shown in Figure 5C is reached. In this way, it is prevented that the sleeve is pressed open too far and as a result of that actually pressing the sleeve closed again is prevented. The notches 58 can moreover have a function in clamping a thin inner lining between their boundaries, when such a lining would be used.

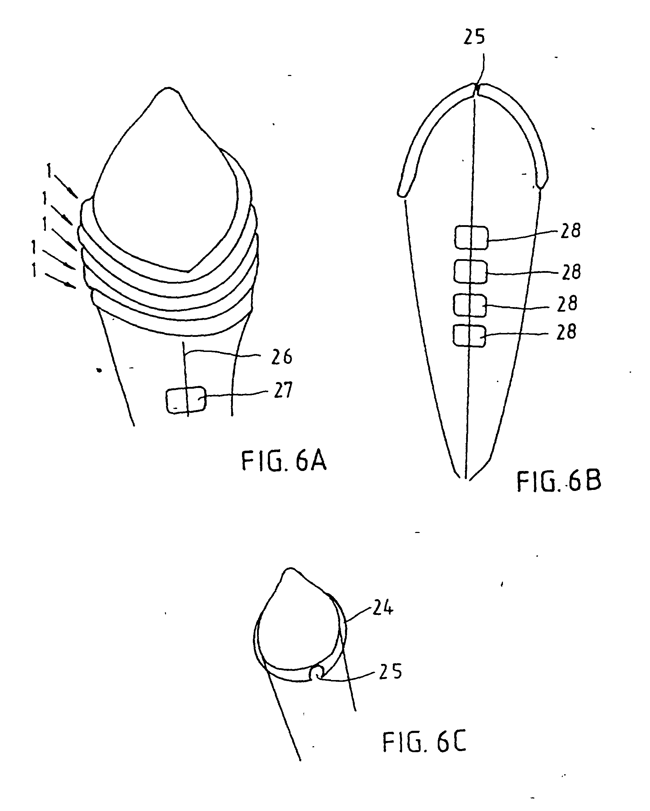

In Figures 6A and 6B similar provisions are schematically shown. In Figure 6A the front side, which is upwardly directed during use, is shown with a folding line 26 and a rough surface area 27 for the thumb. In Figure 6B the folding line 29 is shown and four roughened areas 28 for the four other fingers.

The sleeves 1 shown in Figures 6A and 6B can easily be stacked into one another, but as a result of the folding lines 26 and 29 can also be offered flat, for instance in a vending machine. For folding flat it is provided that the thickened or enlarged upper edge 24 is provided with a notch 25 at the location of the upper end of the folding line 29. A same notch can also be provided in the upper edge 24 at the location of the upper end of the folding line 26. As can be seen in the detail of Figure 6C the interruption in the upper edge 24 forms a means behind which a foil of the inner lining can be hooked or between which it can be clamped.

In particular in case of a moulded upper edge, it can also be alternatively provided with a little knob, on which the inner lining can be held.

In Figure 8 it is schematically shown what the position of the urine sleeve according to the invention during use can be. The urine sleeve 40 has a thickened upper edge 44, over which an upper portion of an inner lining which is not shown has been folded, so that it hangs down with cover flaps 41. The inner lining could possibly, when ready-for-use sleeves are offered be adhered to the upper edge. This adhesion can be facilitated because the broad or thickened upper edge 44 offers a larger engagement surface.

The user P holds the sleeve 40 with one hand, so that the upper edge 44 with the concave portion 44a and the convex portion 44b smoothly abut the surroundings of the vagina. The urine is then released from the urethra 45, so that it can flow downwards, through the sleeve 40, and can flow out of the sleeve 40 in the direction S, so that it seems as if the user urinates like a man, with all the advantages of directing and placing possibilities. The water repellent or waterproof inner lining screens the hands from being wetted by means of the flaps 41. The flaps 41 can possibly run entirely around the sleeve, or just at the front and the rear.

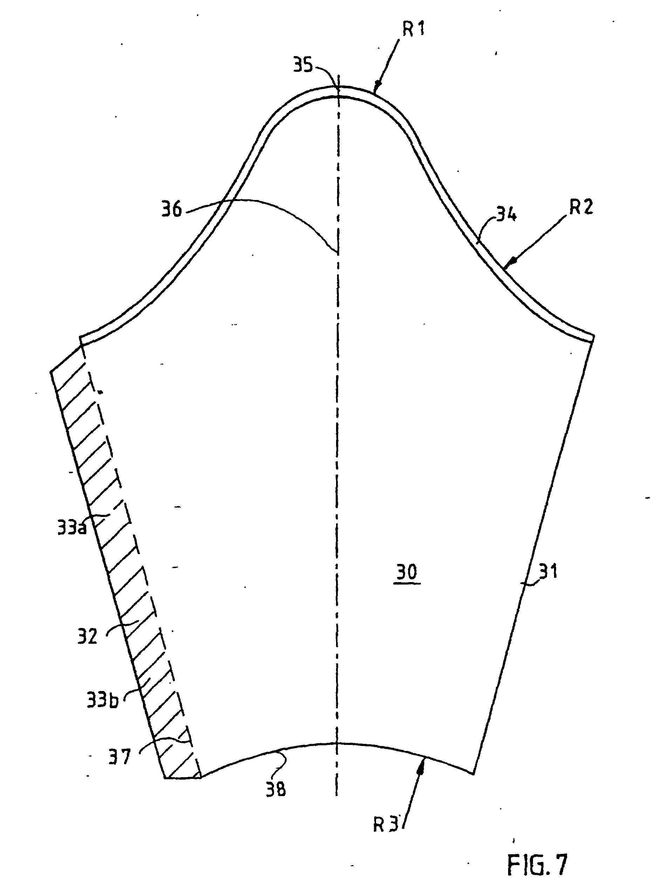

In Figure 7 an embodiment of the urine sleeve according to the invention is shown, which can be offered to the user in flat state. It consists of a plate 30 of easily deformable material, which has an upper edge 34 with a concave portion 34a and a convex portion 34b and a concave lower edge 38. Furthermore, there is a straight side edge 31 and a straight side edge 32, to which latter edge connects an adhesive strip 33a. The adhesive strip 33a is connected to the rest of the plate 30 via folding line 37.

The radius Ri of the convex edge portion 34b is 25 mm in this example. The radius R2 of the concave edge portion 34a is about 113 mm in this example. The radius R3 of the concave lower edge 38 is 133 mm in this example. The distance between the highest point of the lower edge 38 and the highest point of the upper edge 34 is 185 mm in this example, whereas the largest width is about 144 mm, all depending on the user's age.

When assembling the sleeve from the plate 30 the edge 32 is brought to the edge 31 and by moistening or pulling away a protective sheet the adhesive strip 33a is activated to adhere to the surface near the edge 31. Preferably an adhesive strip 33a is provided on both surfaces, to permit folding to both directions at the forming of the sleeve.

After assembling the lines 36 and 37 form folding lines, which can be engaged by the fingers and thumb respectively, to influence the form of the passage in the sleeve.

## Claims

1. Urinating aid for women, comprising a substantially funnel-shaped sleeve (1) with an entrance opening determined by an upper edge (4) of the sleeve (1) and an exit opening determining by a lower edge (3) of the sleeve (1), **characterized** in that the upper edge (4) is provided with one or several elevations (15) that are attached to the upper edge (4), whereby the upper edge (4), in view from aside on the sleeve (1), has a concave portion (4a, 44a) and a convex portion (4b, 44b) that merge fluently in each other, and that the convex portion (4b, 44b) is situated at the rear in the situation of use.

2. Aid according to claim 1, **characterized** in that the concave portion (4a, 44a) and the convex portion (4b, 44b) determine a sinusoidal line.

3. Aid according to claim 1 or 2, **characterized** in that the elevations (15) are elongated and extend in the circumferential direction of the upper edge (4).

4. Aid according to any one of claims 1-3, **characterized** in that the elevations (15) are spaced from one another at a distance which is larger near the concave portion (4a, 44a) than near the convex portion (4b, 44b).

5. Aid according to claim 2, **characterized** in that the elevations (15) near the concave portion (4a, 44a) of the upper edge (4) are shorter than near the convex portion (4b, 44b).

6. Aid according to any one of claims 1-3, **characterized** in that when seen transversely to the sleeve's longitudinal axis, the elevations (15) are narrower than the upper edge (4).

7. Aid according to any one of claims 1-6, **characterized** in that the upper edge (4, 24, 44) is rolled or thickened.

8. Aid according to claim 7, **characterized** in that the upper edge (24) is circumferential with one (25) or several interruptions (58).

9. Aid according to any one of claims 1-8, **characterized** in that the sleeve (1) is provided with longitudinal folding lines (26, 27).

10. Aid according to claim 9, **characterized** in that the longitudinal folding lines (26, 27) are arranged to be situated in a vertical plane during use.

11. Aid according to any one of claims 1-10, **characterized** in that the upper edge (24) forms a thickening or seaming edge having an interruption (25) at the location of a longitudinal folding line (26, 27).

12. Aid according to any one of the preceding claims, **characterized** in that the sleeve (1) is provided with placement means (5, 6) for the fingers of a hand of a user, such as for instance roughened surfaces (27, 28), recesses or recesses limited by elevations.

13. Aid according to claim 12, **characterized** in that the placement means (5, 6) are similarly shaped to either side.

14. Aid according to claim 12 or 13, **characterized** in that the placement means (5, 6) are formed on the sides which during use are on top and below.

15. Aid according to any one of claims 1-14, bin that the sleeve (1) is made of plastic material.

16. Aid according to any one of claims 1-14, **characterized** in that the sleeve (1) is made of cellulose material.

17. Aid according to any one of claims 8-16, **characterized** in that the interruptions (58) in the thickening of the upper edge (4) are formed by inwardly extending substantially V-shaped notches (58), which occur when the sleeve (1) is folded into a flat state.

18. Aid according to any one of claims 1-17 **characterized** in that the sleeve (1) has an upper and a lower portion, the lower portion being movable with respect to the upper portion.

## Patentansprüche

1. Urinierhilfe für Frauen, umfassend eine im wesentlichen trichterförmige Hülse (1) mit einer Eintrittsöffnung, die durch einen oberen Rand (4) der Hülse (1) festgelegt wird, und mit einer durch einen unteren Rand (3) der Hülse (1) festgelegten Austrittsöffnung, **dadurch gekennzeichnet,** daß der obere Rand (4) mit einer oder mehreren Erhebungen (15) ausgestattet ist, die an dem oberen Rand (4) befestigt sind, wodurch der obere Rand (4) in der Seitenansicht der Hülse (1) einen konkaven Abschnitt (4a, 44a) und einen konvexen Abschnitt (4b, 44b) aufweist, die fließend ineinander übergehen, and daß der konvexe Abschnitt (4b, 44b) beim Gebrauch hinten gelegen ist.

2. , Hilfsmittel nach Anspruch 1, **dadurch gekennzeichnet,** daß der konkave Abschnitt (4a, 44a) und der konvexe Abschnitt (4b, 44b) eine sinusförmige Linie bilden.

3. Hilfsmittel nach Anspruch 1 oder 2, **dadurch gekennzeichnet,** daß die Erhebungen (15) länglich sind und sich in Umfangsrichtung des oberen Randes (4) erstrecken.

4. Hilfsmittel nach einem der Ansprüche 1 bis 3, **dadurch gekennzeichnet**, daß daß die Erhebungen (15) voneinander um eine Strecke beabstandet sind, die in der Nähe des konkaven Abschnitts (4a, 44a) größer ist als in der Nähe des konvexen Abschnitts (4b, 44b).

5. Hilfsmittel nach Anspruch 2, **dadurch gekennzeichnet,** daß die Erhebungen (15) nahe dem konkaven Abschnitt (4a, 44a) des oberen Randes (4) kürzer sind als in der Nähe des konvexen Abschnitts (4b, 44b).

6. Hilfsmittel nach einem der Ansprüche 1 bis 3, **dadurch gekennzeichnet,** daß bei Betrachrung quer zu der Längsachse der Hülse die Erhebungen (15) schmaler sind als der obere Rand (4).

7. Hilfsmittel nach einem der Ansprüche 1 bis 6, **dadurch gekennzeichnet,** daß der obere Rand (4, 24, 44) aufgerollt oder verdickt ist.

8. Hilfsmittel nach Anspruch 7, **dadurch gekennzeichnet,** daß der obere Rand (24) sich mit einer (25) oder mehreren Unterbrechungen (58) über den Umfang erstreckt.

9. Hilfsmittel nach einem der Ansprüche 1 bis 8, **dadurch gekennzeichnet,** daß die Hülse (1) mit Längs-Faltlinien (26, 27) ausgestattet ist.

10. Hilfsmittel nach Anspruch 9, **dadurch gekennzeichnet,** daß die Längs-Faltlinien (26, 27) derart angeordnet sind, daß sie bei Benutzung des Hilfsmittels in einer vertikalen Ebene liegen.

11. Hilfsmittel nach einem der Ansprüche 1 bis 10, **dadurch gekennzeichnet,** daß der obere Rand (24) einen Verdickungs- oder Saum-Rand mit einer Unterbrechung (25) an der Stelle einer Längs-Faltlinie (26, 27) bildet.

12. Hilfsmittel nach einem der vorhergehenden Ansprüche, **dadurch gekennzeichnet,** daß die Hülse (1) mit Griffmitteln (5, 6) für die Finger einer Hand einer Benutzerin ausgestattet ist, zum Beispiel in Form aufgerauhter Flächen (27, 28), Ausnehmungen oder durch Erhebungen begrenzter Ausnehmungen.

13. Hilfsmittel nach Anspruch 12, **dadurch gekennzeichnet,** daß die Griffmittel (5, 6) ähnlich auf jeder Seite ausgebildet sind.

14. Hilfsmittel nach Anspruch 12 oder 13, **dadurch gekennzeichnet,** daß die Griffmittel (5, 6) auf den Seiten ausgebildet sind, die bei Benutzung oben und unten liegen.

15. , Hilfsmittel nach einem der Ansprüche 1 bis **4, dadurch gekennzeichnet,** daß die Hülse (1) aus Kunststoff besteht.

16. Hilfsmittel nach einem der Ansprüche 1 bis 14, **dadurch gekennzeichnet,** daß die Hülse (1) aus Zellulosematerial besteht.

17. Hilfsmittel nach einem der Ansprüche 8 bis 16, **dadurch gekennzeichnet,** daß die Unterbrechungen (58) in der Verdickung des oberen Randes (4) durch sich nach innen erstreckende, im wesentlichen V-förmige Kerben (58) gebildet sind, die in Erscheinung treten, wenn die Hülse (1) in einen flachen Zustand gefaltet wird.

18. Hilfsmittel nach einemd er Ansprüche 1 bis 17, **dadurch gekennzeichnet,** daß die Hülse (1) einen oberen und einen unteren Abschnitt besitzt, von denen der untere Abschnitt bezüglich des oberen Abschnitts beweglich ist.

## Revendications

1. Appareil d'aide à la miction pour femmes, comportant un manchon (1) sensiblement en forme d'entonnoir ayant une ouverture d'entrée déterminée par un bord supérieur (4) du manchon (1) et une ouverture de sortie déterminée par un bord inférieur (3) du manchon (1), caractérisé en ce que le bord supérieur (4) est pourvu d'une ou plusieurs surélévations (15) qui sont reliées au bord supérieur (4), grâce à quoi le bord supérieur (4), vu depuis le côté du manchon (1), comporte une partie concave (4a, 44a) et une partie convexe (4b, 44b) qui se rejoignent mutuellement en douceur, et en ce que la partie convexe (4b, 44b) est située en arrière en situation d'utilisation.

2. Appareil d'aide selon la revendication 1, **caractérisé** en ce que la partie concave (4a, 44a) et la partie convexe (4b, 44b) déterminent une ligne sinusoïdale.

3. Appareil d'aide selon la revendication 1 ou 2, **caractérisé** en ce que les surélévations (15) sont allongées et s'étendent dans la direction circonférentielle du bord supérieur (4).

4. Appareil d'aide selon l'une quelconque des revendications 1 à 3, **caractérisé** en ce que les surélévations (15) sont espacées les unes des autres d'une distance qui est plus grande à proximité de la partie concave (4a, 44a) qu'à proximité de la partie convexe (4b, 44b).

5. Appareil d'aide selon la revendication 2, **caractérisé** en ce que les surélévations (15) proches de la partie concave (4a, 44a) du bord supérieur (4) sont plus courtes qu'à proximité de la partie convexe (4b, 44b).

6. Appareil d'aide selon l'une quelconque des revendications 1 à 3, **caractérisé** en ce que, lorsqu'elles sont vues transversalement à l'axe longitudinal du manchon, les surélévations (15) sont plus étroites que le bord supérieur (4).

7. Appareil d'aide selon l'une quelconque des revendications 1 à 6, **caractérisé** en ce que le bord supérieur (4, 24, 44) est roulé ou épaissi.

8. Appareil d'aide selon la revendication 7, **caractérisé** en ce que le bord supérieur (24) est circonférentiel avec une (25) ou plusieurs interruptions (58).

9. Appareil d'aide selon l'une quelconque des revendications 1 à 8, **caractérisé** en ce que le manchon (1) est pourvu de lignes longitudinales (26, 27) de pliage.

10. Appareil d'aide selon la revendication 9, **caractérisé** en ce que les lignes longitudinales de pliage (26, 27) sont agencées de façon à être situées dans un plan vertical pendant l'utilisation.

11. Appareil d'aide selon l'une quelconque des revendications 1 à 10, **caractérisé** en ce que le bord supérieur (24) forme un bord à épaississement ou couture ayant une interruption (25) à l'emplacement d'une ligne longitudinale de pliage (26, 27).

12. Appareil d'aide selon l'une quelconque des revendications précédentes, **caractérisé** en ce que le manchon (1) est pourvu de moyens (5, 6) de positionnement pour les doigts d'une main d'une utilisatrice, tels que, par exemple, des surfaces rugueuses (27, 28), des évidements ou des évidements délimités par des surélévations.

13. Appareil d'aide selon la revendication 12, **caractérisé** en ce que les moyens de positionnement (5, 6) sont de formes similaires sur chaque côté.

14. Appareil d'aide selon la revendication 12 ou 13, **caractérisé** en ce que les moyens de positionnement (5, 6) sont formés sur les côtés qui, pendant l'utilisation, sont situés sur le dessus et le dessous.

15. Appareil d'aide selon l'une quelconque des revendications 1 à 14, **caractérisé** en ce que le manchon (1) est réalisé en matière plastique.

16. Appareil d'aide selon l'une quelconque des revendications 1 à 14, **caractérisé** en ce que le manchon (1) est réalisé en matière cellulosique.

17. Appareil d'aide selon l'une quelconque des revendications 8 à 16, **caractérisé** en ce que les interruptions (58) dans l'épaississement du bord supérieur (4) sont formées par des encoches (58) sensiblement en forme de V s'étendant vers l'intérieur, qui apparaissent lorsque le manchon (1) est plié dans un état à plat.

18. Appareil d'aide selon l'une quelconque des revendications 1 à 17, **caractérisé** en ce que le manchon (1) comporte une partie supérieure et une partie inférieure, la partie inférieure étant mobile par rapport à la partie supérieure.
